# EUROPEAN PATENT APPLICATION

(11) **EP 4 678 220 A1**
(43) Date of publication of application: **14.01.2026**
(21) Application number: 23928586.9
(22) Date of filing: 20.03.2023
(51) Int. Cl.: A61M 60/178

(54) **ANTEGRADE BLOOD-SENDING CANNULA**

(71) Applicant: National University Corporation Kobe University, Kobe-shi, Hyogo 657-8501 (JP)
(72) Inventor: KONISHI, Akihide, Kobe-shi, Hyogo 657-8501 (JP); YAGI, Takahiro, Kusatsu-shi, Shiga 525-8577 (JP)
(74) Representative: Fleck, Hermann-Josef
(86) International application number: PCT/JP2023/010978
(87) International publication number: WO 2024/195003

(57) **Abstract**

Provided is a cannula with which it is possible to effectively and antegradely send blood from the ascending aorta, while reducing the diameter. This cannula, in which the distal end part thereof is disposed in the ascending aorta and blood fed from an external-membrane-type artificial lung is sent antegradely from the ascending aorta, comprises an antegrade blood return part 2 and a blood-sending tube 4, the antegrade blood return part 2 being provided at the distal end of the blood-sending tube 4. A guide wire tube 5 extending in the longitudinal direction from the base end part to the distal end part is disposed substantially at the center of the axis inside the antegrade blood-sending cannula 1. The blood-sending tube 4 is a tube provided on the base end side connected to the extracorporeal-membrane-type artificial lung.

## Description

### [Technical Field]

The present invention relates to a cannula used for an extracorporeal assisted circulation apparatus.

### [Background Art]

When one develops myocardial infarction and the like and his/her cardiac pumping function is sharply degraded, the volume of the left ventricle is excessively increased, causing the contraction of the left ventricle to fail. Under such circumstances, sufficient blood cannot be ejected from the left ventricle so that oxygenated blood cannot be adequately supplied to important organs in the whole body including the brain. In such a case, an intervention by an extracorporeal assisted circulation apparatus is required.

As a percutaneous extracorporeal assisted circulation apparatus currently used, an intracardiac indwelling pump catheter for assisted circulation (IMPELLA (registered trademark) and a percutaneous cardiopulmonary support apparatus (ECMO (PCPS)) are known. Although IMPELLA can reduce the cardiac load, there are the problems that it cannot perform oxygenation and also has strict facility criteria provided.

In contrast, ECMO is broadly used because it can perform oxygenation and has no limitation on facility, but there is the major problem that it increases the cardiac load due to retrograde blood-sending. Further, as a large-diameter cannula must be placed, the problem of the vascular complications associated with the placement of the cannula is also one of the major problems.

First, as to the first problem of an increase in the cardiac load associated with retrograde blood-sending, while ECMO is a device which supplies oxygenated blood to important organs centered on the brain, it sends blood at a high flow rate in the opposite direction to blood which is sent from the heart, resulting in the cardiac load. It can be said that this may prolong recovery of the cardiac function.

Next, with respect to the second problem of the vascular complications associated with the large-diameter cannula, it has been reported that the incidence rate of lower limb ischemia is of the order of 10-70% after the blood-sending tube of ECMO is inserted from the femoral artery. When lower limb ischemia occurs, compartment syndrome and lower limb necrosis may be caused, and reportedly, a patient with complicated lower limb ischemia has a significantly poor prognosis.

According to a meta-analysis focusing on outcomes and complications of ECMO in 1763 adult patients of interest, even with conditions usually associated with a high chance of death, almost 50% of patients receiving ECMO survive up to discharge; on the other hand, patients who die from complications such as bleeding (33%) and sepsis (22%) associated with the long-term placement of the ECMO cannula are not a few (45%) (see Non Patent Literature 1, for example).

In recent years, approaches to the combined use of IMPELLA and ECMO called "ECPELLA" have also been used; according to this, while the cardiac load is alleviated, oxygenated blood can be supplied to the brain. However, there is the problem that there are strict facility criteria as the use of IMPELLA remains unchanged.

For this reason, as a blood-sending cannula used in extracorporeal circulation of blood with a heart-lung machine, a cannula capable of antegradely sending blood is known (see Patent Literature 1). This cannula is composed of an outer tube and an inner tube in which blood is passed through an annular return flow path constructed between the outer tube of which tip end side is blocked and the inner tube, and is ejected from blood-ejecting holes of the outer tube to the aorta in the antegrade direction.

However, with the cannula of Patent Literature 1, although as a mechanism for guiding the blood-ejecting direction to the antegrade direction, inward-facing flange parts are provided at respective hole ends of the blood-ejecting holes, the inward-facing flange parts guide blood in the inner side of the outer tube only, and cannot guide blood ejected from the outer tube. Because of this, there is the problem that guiding in the antegrade direction is not performed sufficiently. In addition, in the cannula of Patent Literature 1, the outer tube is provided so as to cover the inner tube; therefore, there is the problem that a reduction in diameter of the cannula is structurally difficult.

### [Citation List]

### [Patent Literature]

Patent Literature 1: JPA 2015-23970

### [Non-Patent Literature]

Non-Patent Literature 1: Alberto Zangrillo et al., "A meta-analysis of complications and mortality of extracorporeal membrane oxygenation", Crit Care Resusc. 2013 Sep;15(3):172-8.

### [Summary of Invention]

### [Technical Problem]

In view of such circumstances, the present invention aims at providing a cannula capable of effectively and antegradely sending blood from the ascending aorta while promoting a reduction in diameter.

### [Solution to Problem]

In order to solve the above problems, an antegrade blood-sending cannula of the present invention is a cannula of which tip end part is arranged in the ascending aorta and which antegradely sends from the ascending aorta blood supplied from extracorporeal membrane oxygenation, the cannula being composed of: a blood-sending tube provided on the base end side of the cannula and connectable to the extracorporeal membrane oxygenation; and an antegrade blood return part provided on the tip end side of the cannula, wherein the antegrade blood return part comprises: an antegrade blood return mechanism for inverting the blood from the retrograde direction to the antegrade direction to head the blood toward the direction of the base end side; and a blood-sending pressure adjust mechanism for adjusting blood-sending pressure of the blood.

By providing the antegrade blood return part, a collision between blood supplied from extracorporeal membrane oxygenation and blood from the heart can be prevented, and the cardiac load can be reduced effectively. Moreover, by providing the blood-sending pressure adjust mechanism, stimuli to a vascular inner part are reduced and scattered atherosclerotic plaques can be prevented.

Meanwhile, extracorporeal membrane oxygenation herein means an apparatus which oxygenates removed blood extracorporeally and then sends the blood intra-corporeally, and refers to a percutaneous cardiopulmonary support apparatus (ECMO) and the like.

In the antegrade blood-sending cannula of the present invention, the antegrade blood return part is formed with a blood return tube of which tip end is closed and which has at least two side holes on the tip end side, at the inner side of the tip end of the blood return tube, a part of a nearly hemispherical bag body is adhered, and the open end of the bag body is provided in the entire perimeter of the blood return tube including the side holes. And in the antegrade blood return mechanism, the side holes are opened by blood-sending pressure of pressure over a predetermined threshold value, and in the blood-sending pressure adjust mechanism, blood-sending pressure is alleviated by the bag body.

By making the bag body with a structure of which open end is opened and closed, a reduction in diameter of the cannula can be promoted. With regard to the side holes provided in the blood return tube, in order to prevent the blood return tube from unexpectedly moving in the patient's body, it is preferred that when there are two side holes, those are provided at their opposing sites, and when there are more than two side holes, those are provided at nearly uniform intervals around the tube. Pressure over a predetermined threshold value varies depending on the material of the bag body and the size of the side holes. As for the material of the bag body, in the case where an effect of alleviating blood-sending pressure is increased in the blood-sending pressure adjust mechanism, any material having more elasticity can be used. Meanwhile, "the tip end is closed" means that it is closed with regard to the blood flow path, and when a guidewire tube described later is disposed, the tube tip end is opened.

In the antegrade blood-sending cannula of the present invention, in particular, the blood return tube is formed with a first blood return tube provided on the base end side and a second blood return tube provided on the tip end side being fixed by adhesion or thread engagement. The tip end part of the first blood return tube comprises an extension part which is extended with a diameter smaller than that of the base end side. When there is no blood-sending pressure over a predetermined threshold value, the outer peripheral surface of the extension part abuts on and blocks a part of the open end of the bag body, and when there is blood-sending pressure over the predetermined threshold value, an abutment state between the outer peripheral surface of the extension part and the part of the open end of the bag body is opened. In the antegrade blood return mechanism, when the abutment state is opened, antegrade ability is improved due to a flow path formed between the extension part and the part of the open end of the bag body.

By providing the extension part, the flow path formed between the extension part and the part of the open end of the bag body becomes nearly parallel to the flow path before inversion, which improves antegrade ability of blood after inversion. The first blood return tube and the second return tube may not only be fixed by any of adhesion and thread engagement, but also be fixed by the combined use of adhesion and thread engagement to enhance the fixing strength.

In the antegrade blood-sending cannula of the present invention, the antegrade blood return part is formed by extending the blood-sending tube, the antegrade blood return mechanism is shaped by curving the tip end part of the extended blood-sending tube in a U-shape manner, and the blood-sending pressure adjust mechanism may be formed by having at least two side holes formed in the middle part of the extended blood-sending tube. Shaping the tip end part of the blood-sending tube by curving it in a U-shape manner can realize antegrade blood-sending with a more simplified structure.

Further, by providing the side holes in the middle part, blood-sending pressure can be adjusted with a simplified structure. The "U" shape does not mean a strict shape, and it broadly includes curved shapes so that blood flowing through the blood-sending tube is inverted antegradely.

In the antegrade blood-sending cannula of the present invention, in the nearly axial center of the inner part thereof, a guidewire tube is preferably provided which longitudinally extends from the base end part to the tip end part. By providing the guidewire tube, while a reduction in diameter is realized, the guidewire can be inserted into and passed through the inner part of the antegrade blood-sending cannula, which improves convenience. Moreover, the hole of the guidewire tube may be provided along the outer wall of the antegrade blood-sending cannula.

### [Advantageous Effects of Invention]

According to the antegrade blood-sending cannula of the present invention, it has effects of promoting a reduction in diameter and allowing the patient's cardiac load to be alleviated. This can reduce the vascular complications associated with the placement of a large-diameter cannula.

Moreover, according to the antegrade blood-sending cannula of the present invention, it has effects of allowing oxygenated blood to be antegradely sent from the ascending aorta and allowing the blood to be effectively sent to the whole body including the brain.

### [Brief Description of Drawings]

FIG. 1 is an exterior view of an antegrade blood-sending cannula of a first embodiment.
FIG. 2 is a cross-sectional image view of an antegrade blood return part of the first embodiment.
FIG. 3 is an exterior image view of the antegrade blood return part of the first embodiment.
FIG. 4 is an example structure of a second blood return tube.
FIG. 5 is an image view of using the antegrade blood-sending cannula of the present invention.
FIG. 6 is an exterior view of an antegrade blood-sending cannula of a second embodiment.
FIG. 7 is an explanation view of side holes in the middle-stage part.
FIG. 8 is an exterior view of an antegrade blood-sending cannula of a third embodiment.
FIG. 9 is an image view of using a conventional blood-sending cannula.
FIG. 10 is a schematic view of a heartbeat circulation simulator.
FIG. 11 is test results of performance evaluation for the antegrade blood-sending cannula of the first embodiment.

### [Detailed description of Embodiments]

First, an image of using a conventional blood-sending cannula will be explained.

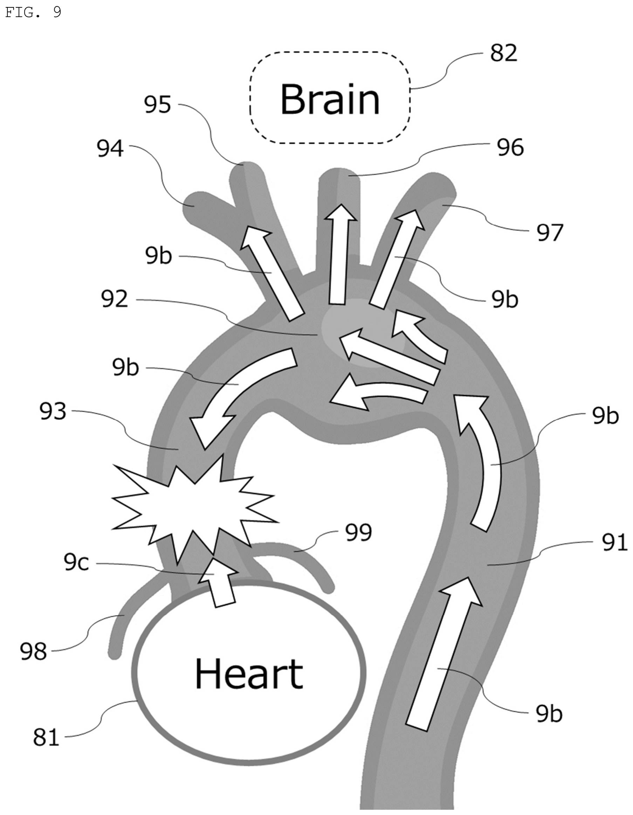

FIG. 9 illustrates the image of using the conventional blood-sending cannula. For the conventional retrograde blood-sending, sufficiently oxygenated and sent blood 9b in extracorporeal membrane oxygenation (not shown) is sent to the descending aorta 91, the aortic arch 92, the right subclavian artery 94, the right common carotid artery 95, the left common carotid artery 96, and the left subclavian artery 97, and then blood 9b is supplied to the brain 82.

However, in the case of the conventional retrograde blood-sending, blood 9b collides with blood 9c which is pumped out of the heart 81 in the position of the ascending aorta 93, thereby causing the cardiac load. Also, due to the collision between the bloodstream from extracorporeal membrane oxygenation and the bloodstream from the heart 81, there is the problem that oxygenated blood 9b from extracorporeal membrane oxygenation is not sufficiently supplied to the brain 82.

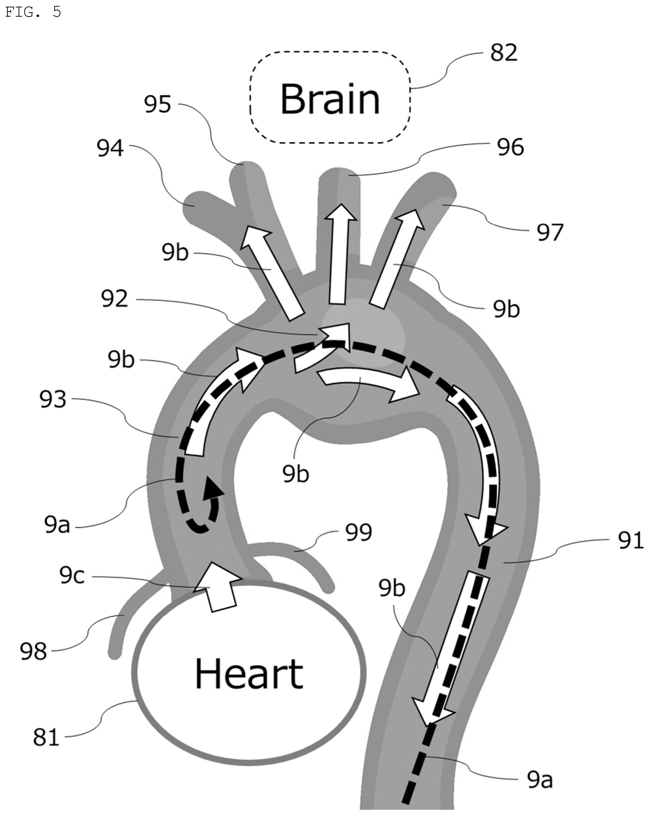

On the other hand, in the antegrade blood-sending cannula of the present invention, as will be explained below, the problem in the conventional retrograde blood-sending is solved. FIG. 5 illustrates an image of using the antegrade blood-sending cannula of the present invention. Here, in FIG. 5, for the sake of explanation, the cannula itself is not shown.

As shown in FIG. 5, for the antegrade blood-sending cannula of the present invention, as the tip end part of the cannula is inserted into and passed through the position of the ascending aorta 93, blood 9a passing through the cannula is sent to the position of the ascending aorta 93, and then is ejected antegradely. Because of this, without colliding with blood 9c pumped out of the heart 81, ejected blood 9b is sent to the aortic arch 92, the descending aorta 91, the right subclavian artery 94, the right common carotid artery 95, the left common carotid artery 96, and the left subclavian artery 97, and then blood 9b is supplied to the brain 82. This can reduce the cardiac load, and results in the oxygenated bloodstream from extracorporeal membrane oxygenation being smoothly supplied to the whole body including the brain 82.

Hereinafter, an example of embodiments of the present invention will be described in detail with reference to the drawings. It is to be noted that the scope of the present invention is not limited to the following embodiments or illustrated examples, and that many modifications and variations can be envisaged.

### [Example 1]

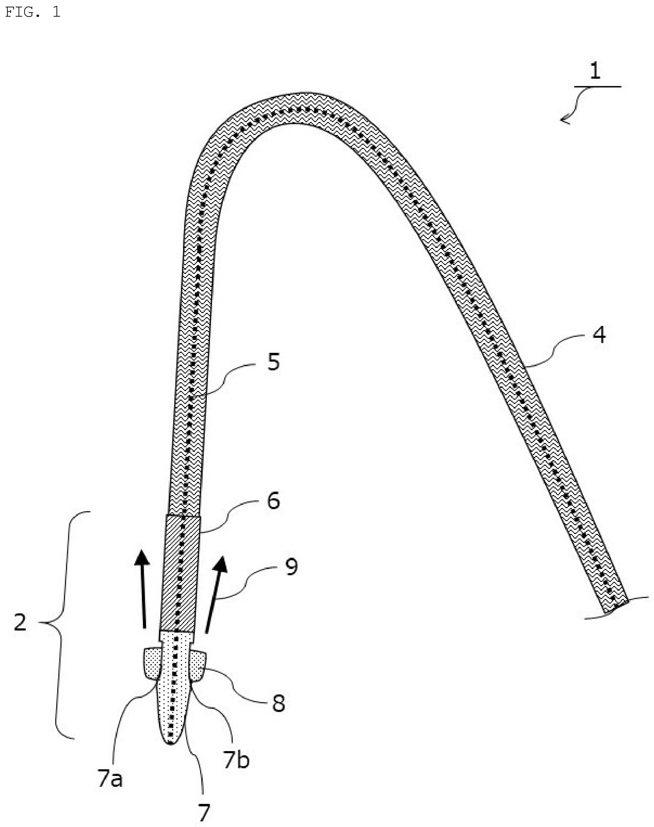

FIG. 1 illustrates an exterior view of an antegrade blood-sending cannula of a first embodiment. In an antegrade blood-sending cannula 1, its tip end part is arranged in the ascending aorta, and blood supplied from extracorporeal membrane oxygenation is antegradely sent from the ascending aorta. The antegrade blood-sending cannula 1 is composed of an antegrade blood return part 2 and a blood-sending tube 4, the antegrade blood return part 2 being provided in the tip end of the blood-sending tube 4. In the nearly axial center of the inner part of the antegrade blood-sending cannula, a guidewire hole is provided which longitudinally extends from the base end part to the tip end part, and a tube 5 is disposed therein.

The blood-sending tube 4 is a tube which is provided on the base end side to be connected to extracorporeal membrane oxygenation, and has an outer diameter of 9 Fr (French catheter scale). When compared with the outer diameter of a blood-sending tube commonly used in extracorporeal membrane oxygenation (about 14-18 Fr), it is understood that a reduction in diameter is promoted. Here, Fr is a unit indicating the outer diameter of tubing such as a catheter, and the outer diameter of the circular catheter of 1 Fr is 1/3 millimeter.

The antegrade blood return part 2 inverts blood from the retrograde direction to the antegrade direction to head it toward the direction of the base end side, and is composed of a first blood return tube 6 provided on the base end side and a second blood return tube 7 provided on the tip end side. In the second blood return tube 7, its tip end is closed and two side holes (7a, 7b) are provided. Also, at the inner side of the tip end of the second blood return tube 7, a part of a nearly hemispherical bag body 8 is adhered. Here, "its tip end is closed" means that it is closed with regard to the blood flow path, and the tip end of the guidewire tube 5 itself is not closed because it does not act as the blood flow path.

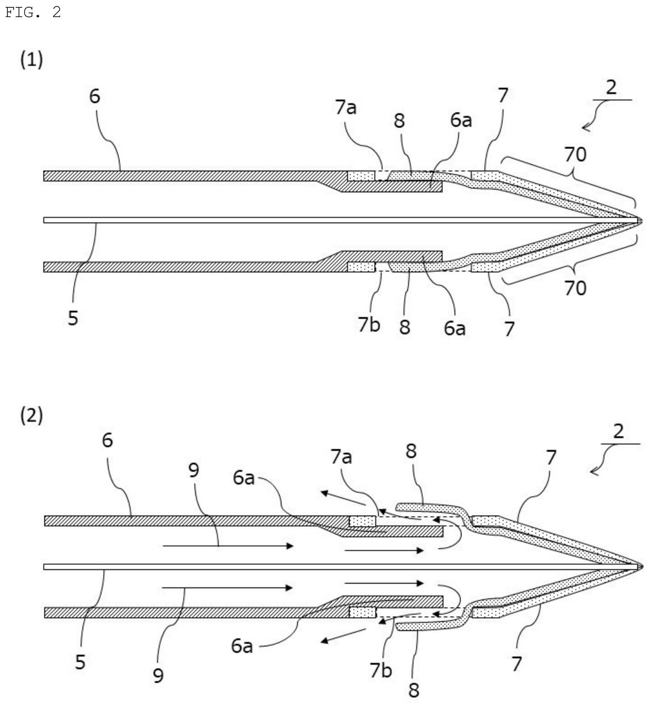

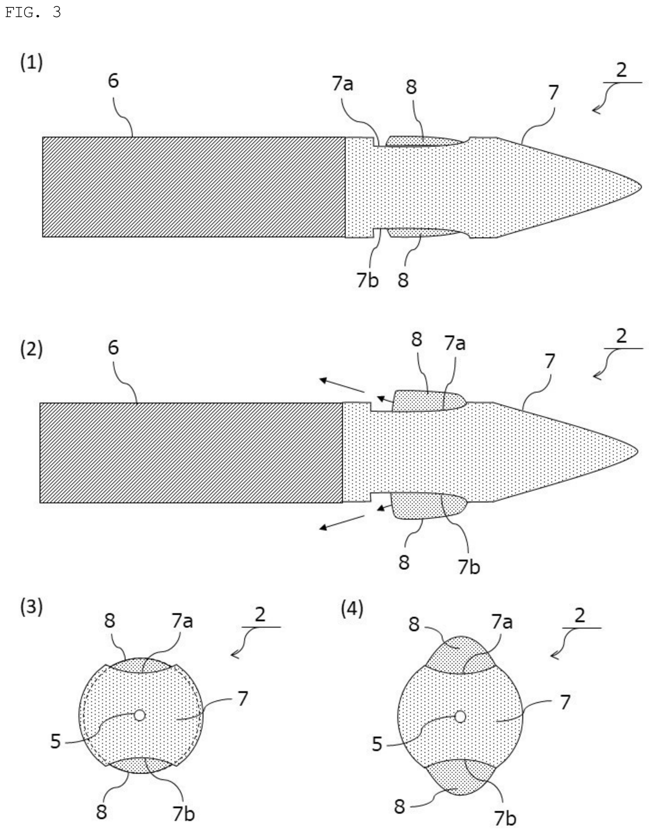

FIG. 2 is a cross-sectional image view of the antegrade blood return cannula in which (1) illustrates a state where blood is not sent and (2) illustrates a state where blood is being sent. In addition, FIG. 3 is an exterior image view of the antegrade blood return cannula in which (1) and (2) illustrate side image views, and (3) and (4) illustrate views viewed from the tip end side. In FIG. 3, (1) and (3) illustrate states where blood is not sent, and (2) and (4) illustrate states where blood is being sent.

As shown in FIG. 2(1), the first blood return tube 6 and the second blood return tube 7 are fitted, adhered and fixed. The first blood return tube 6 has an outer diameter of 9 Fr. Further, a part of the nearly hemispherical bag body 8 is adhered to the inner side of a pointed part 70 of the tip end of the second blood return tube 7. The adhered place may be one place, or may be divided into several places to be adhered. As shown in FIG. 3(3), the open end of the bag body 8 is provided in the entire perimeter of the blood return tube including the side holes (7a, 7b).

As shown in FIG. 2(1), the tip end part of the first blood return tube 6 comprises an extension part 6a which is extended with a smaller diameter than that of the base end side; because of this, when there is no blood-sending pressure over a predetermined threshold value, the outer peripheral surface of the extension part 6a abuts on and blocks a part of the open end of the bag body 8. On the contrary, when there is blood-sending pressure over the predetermined threshold value, as shown in FIG. 2(2), the abutment state between the outer peripheral surface of the extension part 6a and the part of the open end of the bag body 8 is opened.

In the antegrade blood return mechanism, when the abutment state is opened, its antegrade ability is further improved due to the flow path formed between the extension part 6a and the part of the open end of the bag body 8. In particular, as the flow path formed between the extension part 6a and the part of the open end of the bag body 8 becomes nearly parallel to the flow path before inversion, blood 9 after inversion as shown in FIG. 2(2) can be prevented from spreading in the up-down direction, which improves the blood antegrade ability after inversion.

Moreover, as the bag body 8 is formed from silicon, polyurethane, polyvinyl chloride, polyester, natural rubber and the like having high flexibility and elasticity, a degree of opening and closing the open end is automatically adjusted depending on the flow volume and rate of blood 9.

Thus, in the antegrade blood return mechanism in the antegrade blood-sending cannula 1 of the first embodiment, the side holes (7a, 7b) are opened by the blood-sending pressure of pressure over a predetermined threshold value, and in the blood-sending pressure adjust mechanism, the blood-sending pressure is alleviated by the bag body 8. And as shown in FIG. 3(2) or (4), the bag body 8 is opened only when blood is being sent so as to function the antegrade blood return mechanism; because of this, compatibility between antegrade blood-sending and a reduction in diameter is allowed.

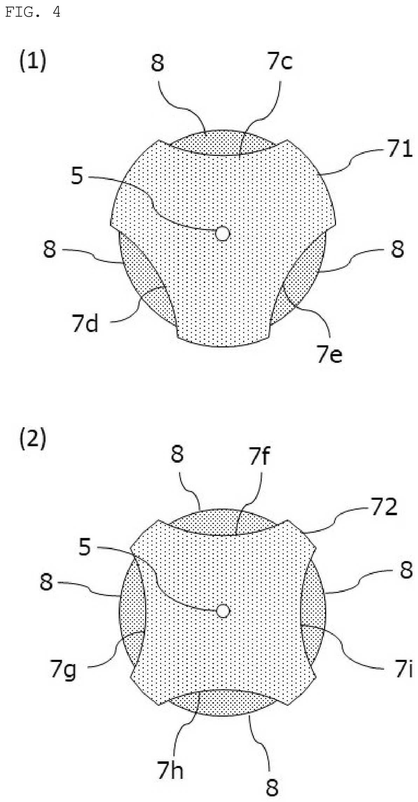

FIG. 4 illustrates an example structure of a second blood return tube. In the antegrade blood-sending cannula 1 of the first embodiment, the second blood return tube 7 is provided with two side holes (7a, 7b). However, as in a second blood return tube 71 shown in FIG. 4(1), it may be the structure that three side holes (7c-7e) are provided, or as in a second blood return tube 72 shown in FIG. 4(2), it may be the structure that four side holes (7f-7i) are provided.

### [Example 2]

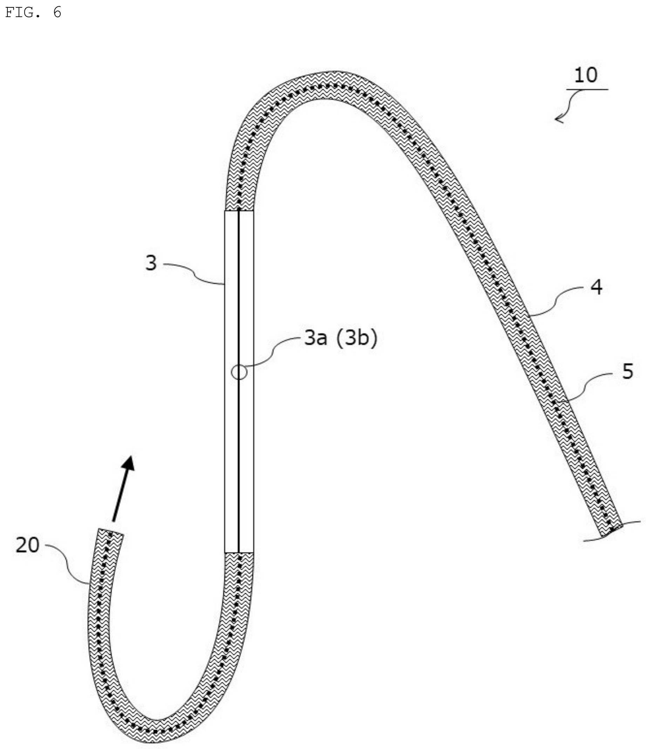

FIG. 6 illustrates an exterior view of an antegrade blood-sending cannula of a second embodiment. An antegrade blood-sending cannula 10 of the second embodiment is composed of blood-sending tubes (3, 4) and an antegrade blood return part 20.

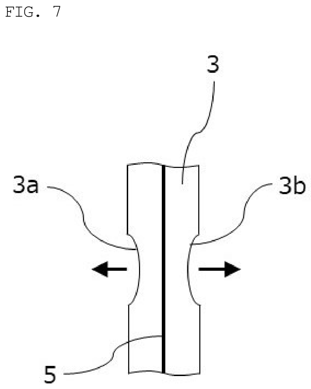

FIG. 7 illustrates an explanation view of side holes in the middle-stage part. As shown in FIG. 7, in the middle part of the blood-sending tube 3, two side holes (3a, 3b) are formed, which can reduce blood-sending pressure.

In the blood-sending tube 3, a material without meshes is used. On the contrary, in the blood-sending tube 4, a material with meshes are used to have resilience. Further, the antegrade blood return part 20 is formed by extending the same material as that of the blood-sending tube 4, and is provided with meshes to hold a curve shape.

In this manner, in the antegrade blood-sending cannula 10 of the second embodiment, the antegrade blood return mechanism is the antegrade blood return part 20 that is shaped by curving the tip end part of the extended blood-sending tube 4 in a U-shape manner, and the blood-sending pressure adjust mechanism is two side holes (3a, 3b) formed in the middle part of the extended blood-sending tube 3.

### [Example 3]

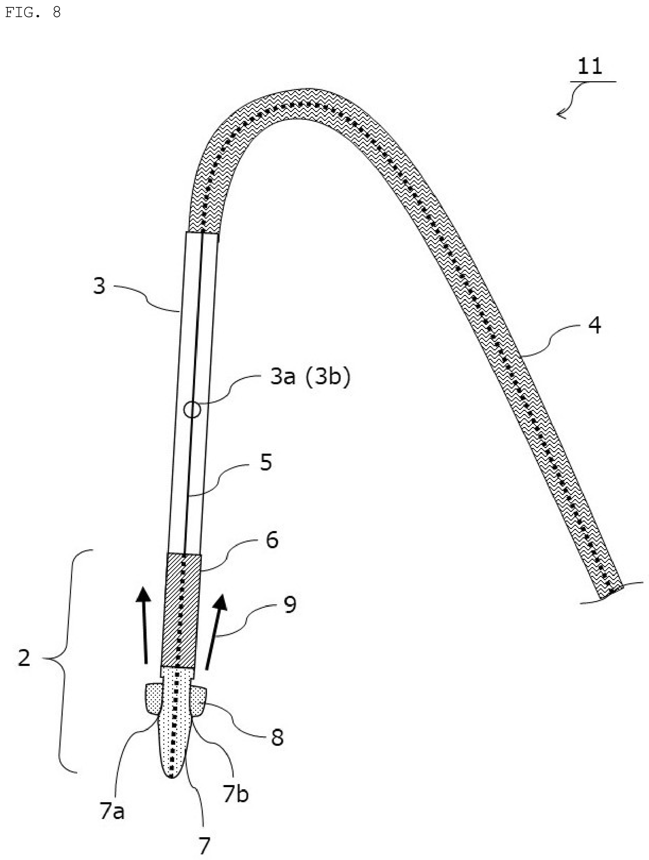

FIG. 8 illustrates an exterior view of an antegrade blood-sending cannula of a third embodiment. In an antegrade blood-sending cannula 11 of the third embodiment, the blood-sending tube 3 in which two side holes (3a, 3b) are formed is provided as in the antegrade blood-sending cannula 10 of the second embodiment. As for the configuration other than that, the antegrade blood-sending cannula 11 is similar to that of the antegrade blood-sending cannula 1 of the first embodiment. By providing the blood-sending tube 3, blood-sending pressure is easily adjusted.

Thus, in the antegrade blood return mechanism in the antegrade blood-sending cannula 11 of the third embodiment, the side holes (7a, 7b) are opened by blood-sending pressure of a pressure over a predetermined threshold value, and in the blood-sending pressure adjust mechanism, blood-sending pressure is alleviated by the bag body 8 and the two side holes (3a, 3b) formed in the middle part of the blood-sending tube 3.

### [Example 4]

A heartbeat circulation simulator (non-clinical test system capable of evaluating a class IV device (highly controlled medical equipment) which was established in Iwasaki Lab, Waseda University) was used to construct a simulated environment in vivo which creates hemodynamics in cardiogenic shock, and moreover, the antegrade blood-sending cannula of Example 1 described above and an existing centrifugal pump were used to compare performance of antegrade blood-sending with that of existing retrograde-sending and quantitatively evaluate how antegrade blood-sending affects the aortic bloodstream and a reduction in cardiac load.

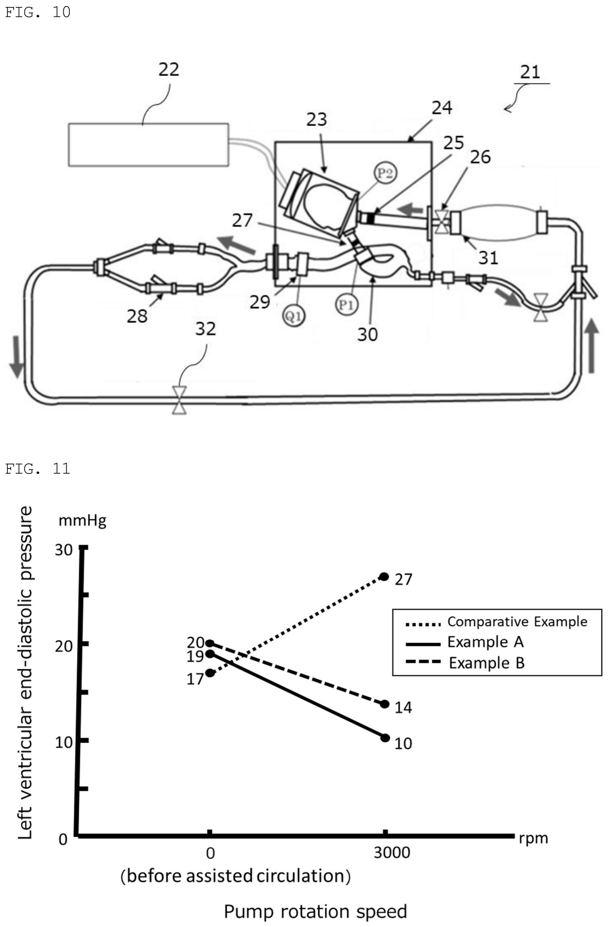

FIG. 10 illustrates a schematic view of the heartbeat circulation simulator which was used in a performance evaluation test. A heartbeat circulation simulator 21 is composed of an air pressure driver 22, a left ventricle model 23, a pressure loading box 24, a mitral valve model 25, a pulmonary vessel resistance 26, an aortic valve model 27, a catheter insertion port 28, an ultrasonic flowmeter 29, an aortic model 30, a venous reservoir tube 31, and a peripheral resistance 32. The flow rate Q₁ [L/min] in the aorta is obtained by the ultrasonic flowmeter 29, and the aortic pressure P₁ and the left ventricular pressure P₂ [mmHg] are obtained by a pressure transducer, respectively. Meanwhile, for the pulmonary vessel resistance 26 and the peripheral resistance 32, the resistance of bloodstream was adjusted by opening and closing valves.

The cannula was inserted from the catheter insertion port 28, and the tip end of the cannula was located near the aortic valve model 27 in the aortic model 30 to perform the test.

In the performance evaluation test of the antegrade blood-sending cannula, antegrade blood-sending cannulas of Example 1 (Examples A, B having different cannula diameters) and a retrograde blood-sending cannula (Comparative Example) were used. Meanwhile, although in the antegrade blood-sending cannula 1 of the Example 1 described above, the blood-sending tube 4 had the outer diameter of 9 Fr, in the antegrade blood-sending cannulas intended for evaluation, the outer diameters of the blood-sending tubes 4 were set to 13 Fr (Example A) and 11 Fr (Example B). The structure other than that is the same as that of Example 1.

In addition, as a retrograde blood-sending cannula of Comparative Example, a blood-sending cannula made by Senko Medical Instrument Mfg. Co., Ltd. (PCKC-A2) was used. The retrograde blood-sending cannula of Comparative Example has an outer diameter of 18 Fr. Meanwhile, as a blood-removal cannula, a blood-removal cannula having a diameter of 18 Fr made by Senko Medical Instrument Mfg. Co., Ltd. (PCKC-V2) was used, and for sending blood, a centrifugal pump and a control unit (CAPIOX SP-101, TERUMO) were used.

As a driving condition, measurement was made before driving the pump (at 0 revolutions per minute [rpm]) and at 3000 rpm. As a test condition of the heartbeat circulation simulator, a heart rate of 70 bpm, an aortic flow rate of 2.8 L/min, and aortic pressure of 70/30 (50) mmHg were determined and created as hemodynamics in cardiogenic shock. Meanwhile, for a working fluid, in order to mimic the viscosity of human blood, an aqueous glycerol solution (20 °C, 33 wt%) in which salt (adjusted to 0.9 wt%) was dissolved was used. Further, the aortic flow rate [L/min] was obtained by the ultrasonic flowmeter, and aortic pressure and left ventricular pressure [mmHg] were obtained by the pressure transducer.

Results of the performance evaluation test for the antegrade blood-sending cannula will be explained with reference to FIG. 11.

When the test results were compared between before driving the pump (at 0 revolutions per minute [rpm]) and at 3000 rpm, it has been seen that while in Comparative Example, left ventricular end-diastolic pressure was increased from 17 mmHg to 27 mmHg, in Example A, left ventricular end-diastolic pressure was decreased from 19 mmHg to 10 mmHg, and in Example B, left ventricular end-diastolic pressure was decreased from 20 mmHg to 14 mmHg.

Further, when Example A in which the blood-sending tube 4 had an outer diameter of 13 Fr and Example B in which the blood-sending tube 4 had an outer diameter of 11 Fr were compared, while in Example B, left ventricular end-diastolic pressure was decreased by 6 mmHg between before driving the pump and at 3000 rpm, in Example A, it was decreased by 9 mmHg. As a result, it has been found out that the blood-sending tube 4 with a larger diameter could more effectively reduce the cardiac load.

Meanwhile, differences in left ventricular end-diastolic pressure of Example A, Example B and Comparative Example before assisted circulation (before driving the pump) are errors in the test.

### [Industrial Applicability]

The present invention is useful as a cannula for use in an extracorporeal assisted circulation apparatus.

### [Reference Signs List]

- 1, 10, 11: Antegrade blood-sending cannula;
- 2, 20: Antegrade blood return part;
- 3, 4: Blood-sending tube;
- 3a, 3b, 7a-7i: Side hole;
- 5: Guidewire tube;
- 6: First blood return tube;
- 6a: Extension part;
- 7, 71, 72: Second blood return tube;
- 8: Bag body;
- 9, 9a-9c: Blood;
- 21: Heartbeat circulation simulator;
- 22: Air pressure driver;
- 23: Left ventricle model;
- 24: Pressure loading box;
- 25: Mitral valve model;
- 26: Pulmonary vessel resistance;
- 27: Aortic valve model;
- 28: Catheter insertion port;
- 29: Ultrasonic flowmeter;
- 30: Aortic model;
- 31: Venous reservoir tube;
- 32: Peripheral resistance;
- 70: Pointed part;
- 81: Heart;
- 82: Brain;
- 91: Descending aorta;
- 92: Aortic arch;
- 93: Ascending aorta;
- 94: Right subclavian artery;
- 95: Right common carotid artery;
- 96: Left common carotid artery;
- 97: Left subclavian artery;
- 98: Right coronary artery;
- 99: Left coronary artery

## Claims

1. A cannula of which tip end part is arranged in the ascending aorta and which antegradely sends from the ascending aorta blood supplied from extracorporeal membrane oxygenation, the cannula is composed of:
a blood-sending tube provided on the base end side of the cannula and connectable to the extracorporeal membrane oxygenation; and
an antegrade blood return part provided on the tip end side of the cannula,
wherein the antegrade blood return part comprises:
an antegrade blood return mechanism for inverting the blood from the retrograde direction to the antegrade direction to head the blood toward the direction of the base end side; and
a blood-sending pressure adjust mechanism for adjusting blood-sending pressure of the blood.

2. The antegrade blood-sending cannula according to claim 1, wherein the antegrade blood return part is formed with a blood return tube of which tip end is closed and which has at least two side holes on the tip end side,
at the inner side of the tip end of the blood return tube, a part of a nearly hemispherical bag body is adhered,
the open end of the bag body is provided in the entire perimeter of the blood return tube including the side holes,
in the antegrade blood return mechanism, the side holes are opened by blood-sending pressure of pressure over a predetermined threshold value, and
in the blood-sending pressure adjust mechanism, blood-sending pressure is alleviated by the bag body.

3. The antegrade blood-sending cannula according to claim 2, wherein the blood return tube is formed with:
a first blood return tube provided on the base end side; and a second blood return tube provided on the tip end side being fixed by adhesion or thread engagement,
the tip end part of the first blood return tube comprises an extension part which is extended with a diameter smaller than that of the base end side,
when there is no blood-sending pressure over a predetermined threshold value, the outer peripheral surface of the extension part abuts on and blocks a part of the open end of the bag body,
when there is blood-sending pressure over the predetermined threshold value, an abutment state between the outer peripheral surface of the extension part and the part of the open end of the bag body is opened, and
in the antegrade blood return mechanism, when the abutment state is opened, antegrade ability is improved due to a flow path formed between the extension part and the part of the open end of the bag body.

4. The antegrade blood-sending cannula according to claim 1, wherein the antegrade blood return part is formed by extending the blood-sending tube,
the antegrade blood return mechanism is shaped by curving the tip end part of the extended blood-sending tube in a U-shape manner, and
the blood-sending pressure adjust mechanism has at least two side holes formed in the middle part of the extended blood-sending tube.

5. The antegrade blood-sending cannula according to claim 3 or 4, wherein in the nearly axial center of the inner part of the antegrade blood-sending cannula, a guidewire tube is provided which longitudinally extends from the base end part to the tip end part.
